(19) **Europäisches Patentamt / European Patent Office / Office européen des brevets**

(11) **EP 4 586 276 A1**

(12) **EUROPEAN PATENT APPLICATION**

(43) Date of publication:
16.07.2025 Bulletin 2025/29

(21) Application number: 25150813.1

(22) Date of filing: 09.01.2025

(51) International Patent Classification (IPC):
*G16H 50/50* (2018.01)    *G16H 30/40* (2018.01)

(52) Cooperative Patent Classification (CPC):
G16H 50/50; G16H 30/40

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 10.01.2024 KR 20240003981

(71) Applicant: POSTECH Research and Business Development Foundation
Pohang-si, Gyeongsangbuk-do 37673 (KR)

(72) Inventors:
• CHOI, Jae Hyun
  37673 Pohang-si, Gyeongsangbuk-do (KR)
• JO, HangJin
  37673 Pohang-si, Gyeongsangbuk-do (KR)
• KIM, Jihun
  37673 Pohang-si, Gyeongsangbuk-do (KR)

(74) Representative: Jung, Minkyu
Wuesthoff & Wuesthoff
Patentanwälte und Rechtsanwalt PartG mbB
Schweigerstraße 2
81541 München (DE)

(54) **METHOD OF OBTAINING OUTLET BOUNDARY CONDITIONS OF BLOOD VESSELS FOR COMPUTATIONAL FLUID DYNAMICS SIMULATION OF BLOOD FLOW WITHOUT IN VIVO BLOOD PRESSURE MEASUREMENT**

(57) The present disclosure relates to a method of obtaining outlet boundary conditions of blood vessels for computational fluid dynamics simulation of blood flow without in vivo blood pressure measurement. Since it is not required to insert a pressure wire, the method can be applied in situations in which it is difficult to obtain in vivo data through invasive methods such as in the cerebral arteries, so it is possible to obtain outlet boundary conditions for performing arterial blood flow simulation without direct in vivo blood pressure measurement. Further, it is possible to quickly obtain optimal conditions in terms of fluid transport energy loss without performing 3D computational fluid dynamics analysis by using the Windkessel model that is a lumped parameter method.

**EP 4 586 276 A1**

## Description

### CROSS-REFERENCE TO RELATED APPLICATIONS

This application claims priority under 35 U.S.C. §119 to Korean Patent Application No. 10-2024-0003981, filed on 01,10, 2024, in the Korean Intellectual Property Office, the disclosure of which is incorporated herein by reference in its entirety.

### TECHNICAL FIELD

The following disclosure relates to a method of obtaining outlet boundary conditions of blood vessels for computational fluid dynamics simulation of blood flow without in vivo blood pressure measurement. In more detail, the present disclosure relates to a method that can obtain outlet boundary conditions for performing computational fluid analysis of blood flow without directly measuring in vivo blood pressure data.

### BACKGROUND

Cerebrovascular disease is a disease with a relatively high morality rate caused by a single disease. In the case of cerebrovascular diseases, the incidence rate in the United States is 85% for ischemic cerebrovascular diseases and 15% for hemorrhagic cerebrovascular diseases. In Korea, cerebral infarction accounts for 43.9%, cerebral hemorrhage for 34.4%, and subarachnoid hemorrhage for 13.2%, with cerebral hemorrhage occurring slightly more frequently. However, the incidence of cerebral infarction has been gradually increasing in recent years. Methods for examining cerebrovascular diseases include angiography, computed tomography (CT), magnetic resonance imaging (MRI), positron emission tomography (PET), etc. Among these, angiography has been widely used to accurately diagnose cerebrovascular diseases, but it is invasive examination procedure and has disadvantages such as the risk associated with the procedure and the time required for the examination.

Accordingly, various studies are being conducted to non-invasively examine cerebrovascular diseases by simulating blood flow using computational fluid dynamics. To simulate blood flow using computational fluid dynamics, blood vessel outlet boundary conditions are essential. In general, flow rate values on the basis of Murray's law or specific pressure functions are imposed as the outlet boundary conditions. To apply Murray's law, the sum of the cubes of the diameters of each daughter blood vessel in a vascular network must be equal to the cube of the diameter of the parent blood vessel. Therefore, when the vascular system to be analyzed does not follow the geometrical requirement of Murray's law, applying flow rate conditions based on this is improper approach because it violates mass conservation. When pressure is assigned as a boundary condition, there are two main methods: assuming 0 Pa and applying pressure functions utilizing Windkessel model. Among these, setting 0 Pa has been shown in research results to lead to inaccurate predictions of flow rate distribution in blood vessel branches during transient state when a blood flow rate changes over time. The method of assigning a specific pressure function utilizing Windkessel model has limitation that actual in vivo blood pressure values are required to calibrate lumped parameters of Windkessel model to determine a blood pressure function over time. This can be applied in cases where in vivo blood pressure measurement is possible through insertion of a pressure wire, as in cardiovascular systems.

Korean Patent No. 10-2099951 (2020. 04. 06) discloses an intracranial pressure measurement device that can more accurately measure intracranial pressure by being positioned as close as possible to the inner wall of a cerebral blood vessel using a stent-shaped intracranial pressure measurement device. However, when inserting a pressure wire directly into the inner wall of a cerebral blood vessel, as with intracranial pressure measurement devices of the related art, there is a risk of unexpected nerve damage or thrombus formation when the blood vessel is small, such as in the case of the cerebral artery.

That is, there is a lack of methodology to appropriately define boundary conditions required for computational fluid analysis of blood vessels, such as cerebral blood vessels where it is difficult to measure pressure data in real blood vessels.

[Related Art Document]

[Patent Document]

Korean Patent No. 10-2099951 (2020. 04. 06.)

**EP 4 586 276 A1**

## SUMMARY

An embodiment of the present disclosure is directed to providing a method of obtaining outlet boundary conditions of blood vessels, the method being able to obtain outlet boundary conditions for performing blood flow simulation without directly measuring in vivo blood pressure and being able to quickly obtain optimal conditions in terms of fluid transport energy loss before performing 3D computational fluid dynamics analysis.

The present disclosure relates to a method of calculating and obtaining outlet boundary conditions of a blood vessel of an examinee using a computer system. In one general aspect, the method of obtaining outlet boundary conditions of blood vessels includes: a biological information providing step of being provided with 3D vascular image data and biological information of the examinee; blood flow information inputting step of obtaining blood flow information from the biological information and inputting the blood flow information as input values; a blood flow equation deriving step of deriving a blood flow equation from the obtained blood flow information; a flow rate function deriving step of deriving a flow rate function for an inflow boundary over time from the blood flow information; a parameter obtaining step of obtaining parameters of a mathematical model of a vascular system by computing simultaneous equations for blood pressure from the blood flow information; and a parameter computing step of computing the parameters using an equation that defines the magnitude of peripheral admittance.

The blood flow equation deriving step may include an equation solution deriving step of deriving a solution of the blood flow equation by applying an integrating factor to the blood flow equation of the blood flow equation deriving step after the blood flow equation deriving step.

The blood flow equation deriving step may include: a boundary condition setting step of setting boundary conditions from the blood flow equation of the blood flow equation deriving step; and a boundary condition blood pressure function deriving step of deriving a blood pressure function of the boundary conditions from the boundary conditions.

The parameter computing step may further include: an integration constant deriving step of deriving an integration constant through a time-averaged flow rate and a pressure equation derived from the mathematical model of the vascular system; a blood pressure function obtaining step of deriving a blood pressure function by substituting the integration constant derived from the time-averaged flow rate and the pressure equation into an equation solution; a parameter equation obtaining step of obtaining parameter equations allowing derivation of parameters by substituting the integration constant into the blood pressure functions of the boundary condition; and a parameter obtaining step of obtaining parameters of the mathematical model of the vascular system from the parameter equations.

The parameter computing step may include an admittance function expression deriving step of deriving an admittance function expression of a blood vessel by applying the mathematical model of the vascular system to 3D vascular image data after the parameter computing step.

The method of obtaining outlet boundary conditions of a blood vessel may further include: after the admittance function expression deriving step, a parameter applying step of applying the parameters to the vascular admittance function expression; and an average flow rate ratio set calculating step of calculating all cases of the average flow rate ratio set over time.

The method of obtaining outlet boundary conditions of a blood vessel may further include a flow rate distribution ratio determining step of determining a value for a flow rate distribution ratio that maximizes the admittance function expression after the average flow rate ratio set calculating step.

The method of obtaining outlet boundary conditions of a blood vessel may further include an outlet pressure function deriving step of deriving an outlet pressure function after the flow rate distribution ratio determining step.

The average flow rate ratio set calculating step may include a time-averaged flow rate ratio set updating step of correcting the time-averaged flow rate ratio set when a convergence condition of the average flow rate ratio set over time is not satisfied in the average flow rate ratio set calculating step, and the time-averaged flow rate ratio set updating step may repeatedly perform the flow rate function deriving step, the parameter obtaining step, the parameter computing step, the admittance function expression deriving step, the parameter applying step, and the average flow rate ratio set calculating step until the flow rate distribution ratio of the average flow rate ratio set over time is determined.

In another general aspect, an apparatus for obtaining outlet boundary conditions of a blood vessel is an apparatus for calculating and obtaining outlet boundary conditions of a blood vessel of an examinee, and the apparatus includes: a processor; and a memory storing a computer program that is executed in the processor, in which the computer program: obtains blood flow information by being provided with 3D vascular image data and biological information of the examinee and derives a blood flow equation by inputting the blood flow information as input values; and derives a flow rate function for an inflow boundary over time from the blood flow information, obtains parameters of a mathematical model of a vascular system by computing simultaneous equations for blood pressure, and computes the parameters using an equation that defines the magnitude of peripheral admittance.

An admittance function expression of a blood vessel may be derived by applying the mathematical model of the vascular system to the 3D vascular image data after the parameters are computed, and the parameters may be applied to the admittance function expression of the blood vessel; and all cases of the average flow rate ratio set over time may be calculated, a value for a flow rate distribution ratio that maximizes the admittance function expression may be calculated, and an outlet pressure function may be derived.

When a convergence condition of the average flow rate ratio set over time is not satisfied, parameters and an admittance function expression may be derived by deriving a flow rate function until a convergence condition of the average flow rate ratio set over time is satisfied by correcting the time-averaged flow rate ratio set, and calculation of an average flow rate ratio set may be repeatedly performed by applying the derived parameters.

## BRIEF DESCRIPTION OF THE DRAWINGS

FIG. 1 is a schematic diagram of a two-element Windkessel model.
FIG. 2 is a schematic diagram of a cerebral artery network system with a two-element Windkessel model applied at the outlet of a blood vessel.
FIG. 3 is a diagram showing the steps of a method of obtaining outlet boundary conditions of a blood vessel of the present disclosure.

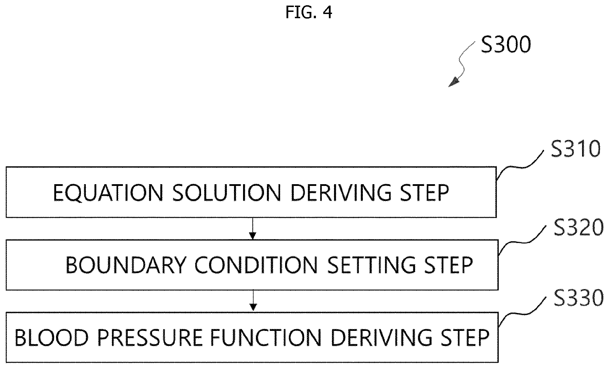

FIG. 4 is a diagram showing the detailed steps of a blood flow equation derivation step of the present disclosure.

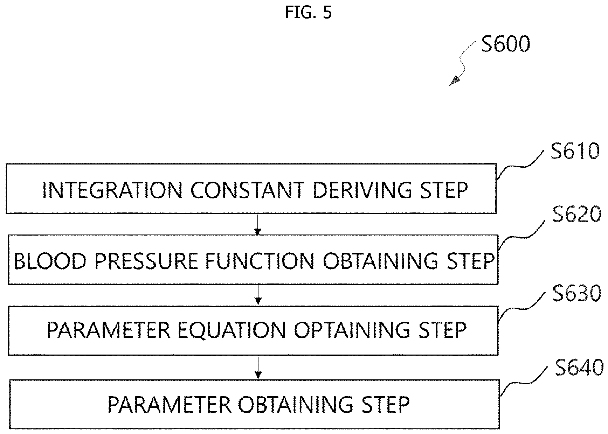

FIG. 5 is a diagram showing the detailed steps of a parameter computing step of the present disclosure.
FIG. 6 is a contour diagram of the dimensionless peripheral admittance magnitude derived by the method of obtaining outlet boundary conditions of a blood vessel of the present disclosure.
FIG. 7 is a graph of a cerebral artery outlet pressure function derived by the method of obtaining outlet boundary conditions of a blood vessel of the present disclosure.

## DETAILED DESCRIPTION OF EMBODIMENTS

Embodiments of the present disclosure will be described hereafter in detail with reference to the accompanying drawings. However, these are only examples and the present disclosure is not limited to the detailed embodiments described in the examples.

Unless defined otherwise, all of technical terms and scientific terms have the same meanings as the meanings that are generally understood by one of those skilled in the art. The terms used to describe the present disclosure is provided to only effectively describe specific detailed examples and is not intended to limit the present disclosure.

Further, the similar forms used in the specification and the claims may be intended to include plurality forms unless there is a specific instruction in the context. Further, unless explicitly described otherwise, "comprising" any components will be understood to imply the inclusion of other components rather than the exclusion of any other components.

The Windkessel model, which is used to derive boundary conditions of a blood vessel using a blood vessel pressure measurement value, explains the relationship between blood pressure and a blood flow rate on the basis of the similarity between a fluid transport system and an electrical circuit system. Lumped parameters of the Windkessel model that is a mathematical model of a vascular system need to be determined to obtain a blood pressure function over time. Accordingly, in the present disclosure, it has been made possible to obtain a blood pressure function without in vivo blood pressure measurement from commonly known systolic blood pressure and diastolic blood pressure information. When analyzing flow in a network composed of multiple blood vessels, flow rate information at the branches of each blood vessel is needed to obtain outlet pressure boundary conditions of each blood vessel. By calculating the peripheral admittance magnitude while changing the flow rate distribution ratio at each branching point, a blood vessel outlet pressure function is derived from the flow rate distribution ratio that results in the largest admittance. In this case, the admittance is the reciprocal of impedance, so the physical meaning of maximum admittance is that the energy consumed in transporting blood through a vascular network is minimized. Accordingly, even though it is difficult to directly measure the pressure in blood vessels such as brain vasculature, it is possible to derive appropriate pressure boundary conditions for computational fluid analysis.

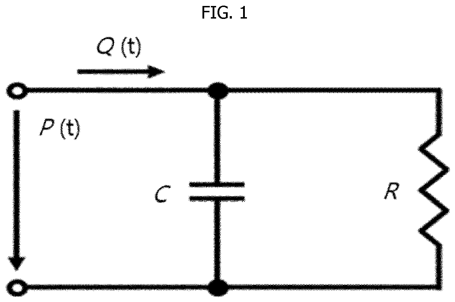

In the present disclosure, a method of obtaining a blood pressure function by applying a two-element Windkessel model that is the mathematic model of a blood vessel system as in FIG. 1 was adopted. FIG. 1 is a schematic diagram of a two-element Windkessel model, in which the two-element Windkessel model that is a mathematical model of a vascular system means modeling a vascular system with two main components or elements and is primarily used to describe and predict blood pressure and blood flow. Accordingly, in the present disclosure, a two-element Windkessel model that is the simplest form was adopted to apply the Windkessel model in the absence of in vivo blood pressure data. In this case, in order to utilize the Windkessel model that is a mathematical model of a vascular system, it is required to obtain model parameter values given as R (resistance) and C (compliance) values. It is possible to derive a pressure waveform from the model parameter values. Accordingly, it is possible to obtain a blood pressure function without direct in vivo blood pressure measurement using systolic blood pressure and diastolic blood pressure information.

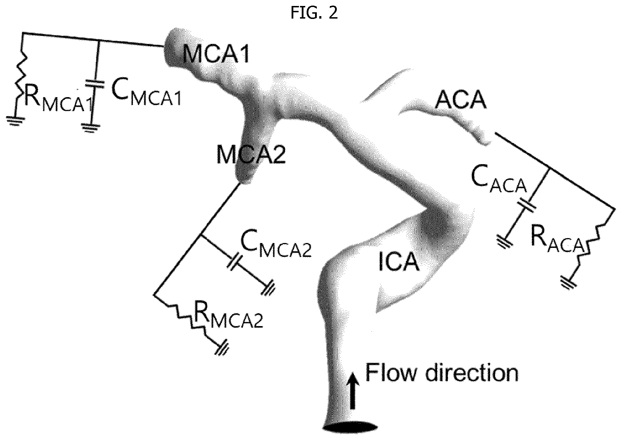

Further, FIG. 2 is a schematic diagram of a cerebral artery network system with a two-element Windkessel model applied at the outlets of blood vessels. It is required to analyze flow in a network having multiple blood vessels, but the system composed of a vascular bundle is a parallel system, as in FIG. 2, so the concept of admittance has been introduced in the present disclosure when determining parameters of the Windkessel model that is a mathematical model of a vascular system. In this case, the admittance is a parameter that represents how well a current flows in an electrical circuit. The present disclosure proposes a method of determining model parameters of the Windkessel model that is a mathematical model of a vascular system on the basis of the condition where admittance is maximized, that is, the condition where blood flows is optimal in terms of energy loss. Further, referring to FIG. 2, it can be seen that ACA, MCA1, and MCA2 that are branching points of three blood vessels are in a parallel connection state. In this case, since the admittance is defined as the reciprocal of impedance, it is possible to simply derive the total impedance of a vascular system by applying the relationship between the admittance and the impedance in analysis of the parallel system of blood vessels.

Next, a method of obtaining outlet boundary conditions of blood vessels for computational fluid dynamics simulation of blood flow without in vivo blood pressure measurement of the present disclosure is described in detail with reference to the drawings.

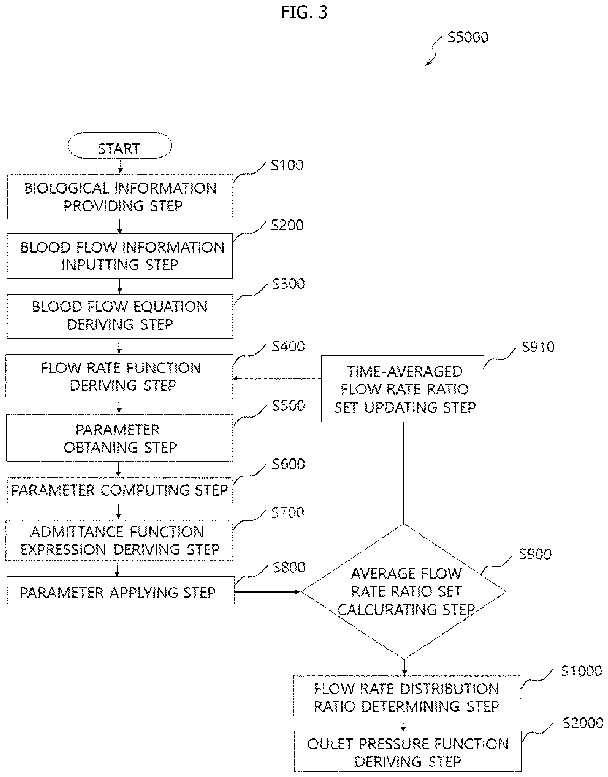

FIG. 3 is a diagram showing the steps of a method of obtaining outlet boundary conditions of a blood vessel of the present disclosure. Referring to FIG. 3, the method of obtaining outlet boundary conditions of a blood vessel of the present disclosure includes a biological information providing step S100, a blood flow information inputting step S200, a blood flow equation deriving step S300, a flow rate function deriving step S400, a parameter obtaining step S500, and a parameter computing step S600.

The biological information providing step S100 is a step of being provided with 3D vascular image data and biological information. The 3D vascular image is obtained through MRI, CT, 3D ultrasound, etc., and includes the ICA (internal carotid artery), MCA (middle cerebral artery), and ACA (anterior cerebral artery). Further, it is possible to be provided with biological information from the 3D vascular image data and the biological information can be obtained by visualizing information about the brain, muscles, joints, bones, blood vessels, etc.

The blood flow information inputting step S200 is a step of obtaining blood flow information from the biological information and inputting the blood flow information as input values (input values: $Q_{ICA}$, $P_S$, $P_D$, $Q_{ACA}$, $Q_{MCA}$). In detail, the blood flow information includes $Q(t)$ (an arbitrary blood flow rate function), Ps (systolic blood pressure), $P_D$ (diastolic blood pressure), and the flow rate distribution ratio of a blood vessel. Referring to FIG. 2, the system composed of a vascular bundle is a parallel system, so the vascular bundle is divided into ICA, ACA, MCA1, and MCA2. Since branching of blood vessels occurs twice in FIG. 2, two sets consisting of $\overline{Q}_{ACA}/\overline{Q}_{MCA}$ and $\overline{Q}_{MAC2}/\overline{Q}_{MCA1}$ can be set as input values. The blood flow equation deriving step S300 is a step of deriving a blood flow equation (Equation (1)) from the obtained blood flow information. $Q(t)$ (arbitrary blood flow rate function) can be derived from Q (an arbitrary blood flow rate) of the blood flow information. In this case, it is possible to derive a blood flow equation, which is a governing equation for $Q(t)$, as Equation (1) by applying Kirchhoff's law to the two-element Windkessel model circuit of FIG. 1.

$$\text{(Equation 1)}$$

$$C\frac{dP(t)}{dt} + \frac{1}{R}P(t) = Q(t)$$

Further, the method of obtaining outlet boundary conditions of a blood vessel includes an equation solution deriving step S310 of deriving the solution of a blood flow equation by applying an integrating factor to Equation (1) that is the blood flow equation derived in the blood flow equation deriving step S300. A blood flow equation solution can be derived as in Equation (3) by multiplying both sides of Equation (1) by the integrating factor $e^{t/(RC)}$ and integrating.

$$\text{(Equation 3)}$$

$$P(t) = a_0 R + \sum_{n=1}^{\infty}\left[\frac{(T^2 R a_n - 2n\pi T R^2 C b_n)\cos\left(\frac{2n\pi t}{T}\right) + (2n\pi T R^2 C a_n + T^2 R b_n)\sin\left(\frac{2n\pi t}{T}\right)}{T^2 + (2n\pi RC)^2}\right] + k e^{\frac{-t}{RC}}$$

Further, the method of obtaining outlet boundary conditions of a blood vessel includes a boundary condition setting step S320 and a blood pressure function deriving step S330 after the blood flow equation deriving step S300.

The boundary condition setting step S320 is a step of setting boundary conditions (Equation (1.1) and Equation (1.2)) for the blood flow equation of the blood flow equation deriving step S300. The boundary conditions can be derived as in Equation (1.1) and Equation (1.2) for diastolic blood pressure and systolic blood pressure information, respectively.

(Equation 1.1)

$$P = P_d \text{ at } t = (q-1)T$$

(Equation 1.2)

$$P = P_s \text{ at } t = (q-1)T + t_s$$

The blood pressure function deriving step S330 is a step of deriving blood pressure functions (Equation (4) and Equation (5)) from the boundary conditions (Equation (1.1) and Equation (1.2)). In this case, Equation (4) and Equation (5) that are blood pressure functions of the boundary conditions can be derived by applying Equation (1.1) and Equation (1.2) that are the boundary conditions to Equation (3).

(Equation 4)

$$k = \left[ P_d - a_0 R - TR \sum_{n=1}^{\infty} \left( \frac{Ta_n - 2n\pi RCb_n}{T^2 + (2n\pi RC)^2} \right) \right] e^{\frac{(q-1)T}{RC}}$$

(Equation 5)

$$P_s = a_0 R + \sum_{q=1}^{\infty} \left[ \frac{(T^2 Ra_q - 2n\pi TR^2 Cb_q)\cos\left(\frac{2n\pi t_s}{T}\right) + (2n\pi TR^2 Ca_q + T^2 Rb_q)\sin\left(\frac{2n\pi t_s}{T}\right)}{T^2 + (2n\pi RC)^2} \right] + ke^{\frac{-[(q-1)T + t_s]}{RC}}$$

The flow rate function deriving step S400 is a step of deriving a flow rate function for an inflow boundary over time from the blood flow information. In detail, the flow rate function Q(t) included in the blood flow equation derived in the blood flow equation deriving step S300 can be derived as a flow rate function for the inflow boundary using Fourier series, as in Equation (2).

(Equation 2)

$$Q(t) = a_0 + \sum_{n=1}^{\infty} \left[ a_n \cos\left(\frac{2n\pi t}{T}\right) + b_n \sin\left(\frac{2n\pi t}{T}\right) \right]$$

The parameter obtaining step S500 is a step of obtaining lumped parameters R and C of a mathematical model of a vascular system by computing simultaneous equations for blood pressure (Equation (8) and Equation (9)) from the blood flow information.

The parameter computing step S600 is a step of computing the lumped parameters R and C using an equation that defines the magnitude of peripheral admittance. In detail, the parameter computing step further includes an integration constant deriving step S610, a blood pressure function obtaining step S620, a parameter equation obtaining step S630, and a parameter obtaining step S640.

The integration constant deriving step S610 is a step of deriving an integration constant through a time-averaged flow rate and a pressure equation (Equation (6)) derived from a mathematical model of a vascular system. In detail, because resistance is the ratio of time-averaged pressure to time-averaged flow rate in two-element Windkessel model that is a mathematical model of a vascular system, an integration constant k = 0 can be derived from Equation (6).

$$(\text{Equation } 6)$$

$$R = \frac{\int_0^T P(t)dt/T}{\int_0^T Q(t)dt/T} = R + \frac{kRC}{Ta_0}\left(1 - e^{\frac{-T}{RC}}\right)$$

The blood pressure function obtaining step S620 is a step of obtaining a blood pressure function (Equation (7)) by substituting the integration constant derived from the time-averaged pressure to flow rate ratio (Equation (6)) into the equation solution (Equation (3)). An analytical solution of the two-element Windkessel model that is a mathematical model of a vascular system can be derived as Equation (7) that is a blood pressure function from the integration constant derived from the integration constant deriving step S610.

$$(\text{Equation } 7)$$

$$P(t) = a_0 R + TR \sum_{n=1}^{\infty} \left[ \frac{(Ta_n - 2n\pi RC b_n)\cos\left(\frac{2n\pi t}{T}\right) + (2n\pi RC a_n + Tb_n)\sin\left(\frac{2n\pi t}{T}\right)}{T^2 + (2n\pi RC)^2} \right]$$

The parameter equation obtaining step S630 is a step of obtaining parameter equations (Equation (8) and Equation (9)) by substituting the integration constant into the blood pressure functions (Equation (4) and Equation (5)) of the boundary conditions.

$$(\text{Equation } 8)$$

$$P_s = a_0 R + TR \sum_{n=1}^{\infty} \left[ \frac{(Ta_n - 2n\pi RC b_n)\cos\left(\frac{2n\pi t_s}{T}\right) + (2n\pi RC a_n + Tb_n)\sin\left(\frac{2n\pi t_s}{T}\right)}{T^2 + (2n\pi RC)^2} \right]$$

$$(\text{Equation } 9)$$

$$P_d = a_0 R + TR \sum_{n=1}^{\infty} \left[ \frac{Ta_n - 2n\pi RC b_n}{T^2 + (2n\pi RC)^2} \right]$$

The parameter obtaining step S640 is a step of obtaining lumped parameters R and C of a mathematical model of a vascular system from the parameter equations (Equation (8) and Equation (9)). Because $a_n$ and $b_n$ that are Fourier coefficients in the parameter equations (Equation (8) and Equation (9)) are determined from a blood flow rate function (Equation (2)), it is possible to obtain lumped parameters R and C of the Windkessel model that is a mathematical model of a vascular system by introducing values for systolic and diastolic blood pressures and simultaneously solving the parameter equations (Equation (8) and Equation (9)). In this case, the blood flow rate function can be obtained from phase-contrast magnetic resonance imaging (PC-MRI), so a blood pressure function over time can be obtained from Equation (7) without direct in vivo blood pressure measurement.

The method of obtaining outlet boundary conditions of a blood vessel of the present disclosure further includes an admittance function expression deriving step S700 after the parameter computing step S600. The admittance function expression deriving step S700 is a step of deriving an admittance function expression (Equation (10)) of a blood vessel by applying a mathematical model of a vascular system to 3D vascular image data. In detail, in the cerebral arterial network system composed of the internal carotid artery, the middle cerebral artery, and the anterior cerebral artery shown in FIG. 2, the magnitude of the peripheral admittance is defined by a vascular admittance function expression (Equation (10)).

The method of obtaining outlet boundary conditions of a blood vessel of the present disclosure includes a parameter applying step S800 and an average flow rate ratio set calculating step S900 after the admittance function expression deriving step.

The parameter applying step S800 is a step of applying the lumped parameters R and C to the vascular admittance function expression (Equation (10)).

(Equation 10)

$$\|Y_{ph}\| = \sqrt{\left(\frac{1}{R_{ACA}} + \frac{1}{R_{MCA1}} + \frac{1}{R_{MCA2}}\right)^2 + \omega^2 (C_{ACA} + C_{MCA1} + C_{MCA2})^2}$$

Referring to FIG. 2, when a network is composed of multiple branches of arteries, that is, arteries are formed in a parallel structure, it is required to determine first a flow rate distribution ratio at which an admittance value is maximum by introducing the admittance value. In this case, the flow rate distribution ratio is substituted into $a_0$ that is a time-averaged flow rate value in Equation (8) and Equation (9). Accordingly, it is possible to obtain $a_0$ at which $\|Y_{ph}\|$ that is an admittance value of a vascular network is the largest.

The average flow rate ratio set calculating step S900 is a step of calculating all cases of the average flow rate ratio set over time. It is possible to determine lumped parameters R and C of the Windkessel model that is a mathematical model of a vascular system by simultaneously solving Ps and $P_D$ from Equation (8) and Equation (9), and accordingly, it is possible to obtain R and C values at the outlet of each of ACA, MCA1, and MCA2.

Further, the method includes a flow rate distribution ratio determining step S1000 after the average flow rate ratio set calculating step S900. The flow rate distribution ratio determining step S1000 is a step of determining a value for a flow rate distribution ratio that maximizes Equation (10) that is an admittance function expression.

Further, the method includes an outlet pressure function deriving step S2000 after the flow rate distribution ratio determining step S1000. The outlet pressure function deriving step S2000 is a step of determining a flow rate distribution ratio at which peripheral admittance is maximum in an arterial network system having multiple blood vessels and of obtaining outlet pressure functions ACA, MCA1, and MCA2 of respective blood vessels in the condition.

Further, the method further includes a time-averaged flow rate ratio set updating step S910 of correcting a time-averaged flow rate ratio set when all cases of average flow rate ratio set for time are not calculated in the average flow rate ratio set calculating step S900. In this case, the process of simultaneously calculating Ps and $P_D$ from Equation (8) and Equation (9) is again performed. Accordingly, it is possible to determine a flow rate distribution ratio that maximizes the admittance value and it is possible to output outlet pressure functions for ACA, MCA1, and MCA2 from the flow rate distribution ratio.

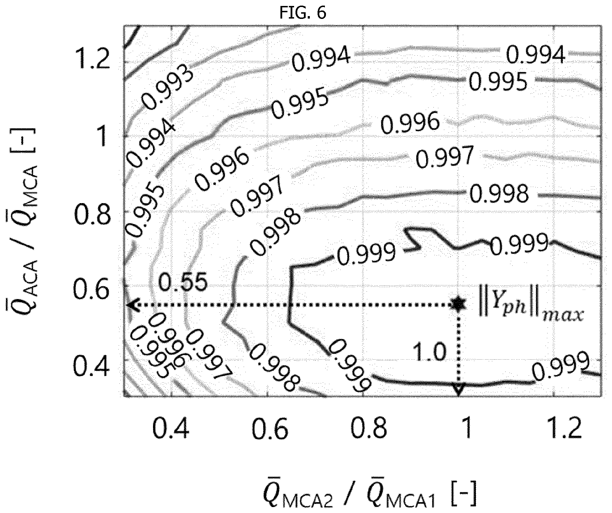

FIG. 6 is a contour diagram of the dimensionless peripheral admittance magnitude derived by the method of obtaining outlet boundary conditions of a blood vessel of the present disclosure. Referring to FIG. 6, the x axis is the flow rate distribution ratio of MCA2 and MCA1 and y axis is the flow rate distribution ratio of ACA and MCA. In this case, a value corresponding to the point where the admittance value of the peripheral region of a blood vessel is maximized is output.

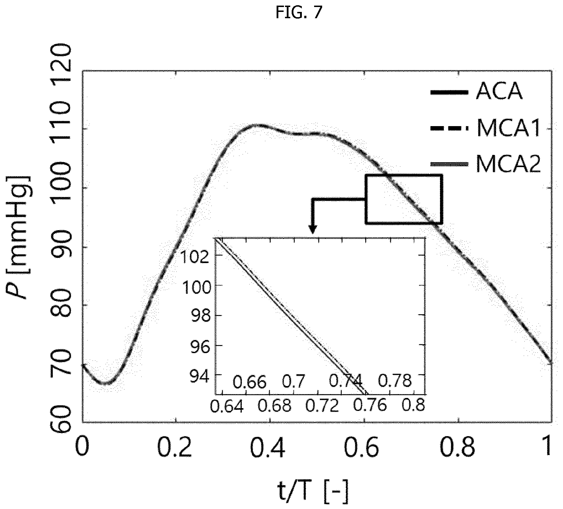

Further, FIG. 7 is a graph of a cerebral artery pressure function derived by the method of obtaining outlet boundary conditions of a blood vessel of the present disclosure, and it is possible to derive a cerebrovascular outlet pressure function as in FIG. 7 with reference to FIG. 6. Cerebral artery blood pressure may be higher or lower than cardiac blood pressure at the same time due to temporal variations. However, it was assumed that the time-averaged flow rate flows to maximize the admittance of the cerebrovascular network such that a blood vessel outlet pressure function can be derived.

Accordingly, it is possible to obtain outlet boundary conditions for performing arterial blood flow simulation without direct in vivo blood pressure measurement through the method of obtaining outlet boundary conditions of blood vessels for computational fluid dynamics simulation of blood flow without in vivo blood pressure measurement of the present disclosure. Further, since it is not required to insert a pressure wire, the method can be applied in situations in which it is difficult to obtain in vivo data through invasive methods such as in the cerebral arteries. Further, it is possible to quickly obtain optimal conditions in terms of fluid transport energy loss without performing 3D computational fluid dynamics analysis by using the Windkessel model that is a lumped parameter method.

According to the present disclosure, it is possible to obtain vascular outlet boundary conditions for performing blood flow simulation without direct in vivo blood pressure measurement.

Further, according to the present disclosure, since it is not required to insert a pressure wire, the method can be applied in situations in which it is difficult to obtain in vivo data through invasive methods such as in the cerebral arteries.

Further, according to the present disclosure, it is possible to quickly obtain optimal conditions in terms of fluid transport energy loss without performing 3D computational fluid dynamics analysis by using the Windkessel model that is a lumped parameter method.

The above description is only an example to which the principle of the present disclosure has been applied and other components may be further included without departing from the scope of the present disclosure.

**EP 4 586 276 A1**

**Claims**

1. A method of calculating and obtaining outlet boundary conditions of a blood vessel of an examinee using a computer system, the method comprising:

   a biological information providing step of being provided with 3D vascular image data and biological information of the examinee;
   a blood flow information inputting step of obtaining blood flow information from the biological information and inputting the blood flow information as input values;
   a blood flow equation deriving step of deriving a blood flow equation from the obtained blood flow information;
   a flow rate function deriving step of deriving a flow rate function for an inflow boundary over time from the blood flow information;
   a parameter obtaining step of obtaining parameters of a mathematical model of a vascular system by computing simultaneous equations for blood pressure from the blood flow information; and
   a parameter computing step of computing the lumped parameters using an equation that defines the magnitude of peripheral admittance.

2. The method of claim 1, wherein the blood flow equation deriving step includes an equation solution deriving step of deriving a solution of the blood flow equation by applying an integrating factor to the blood flow equation of the blood flow equation deriving step after the blood flow equation deriving step.

3. The method of claim 1 or 2, wherein the blood flow equation deriving step includes:

   a boundary condition setting step of setting boundary conditions from the blood flow equation of the blood flow equation deriving step; and
   a boundary condition blood pressure function deriving step of deriving a blood pressure function of the boundary conditions from the boundary conditions.

4. The method of any one of claims 1 to 3, wherein the parameter computing step further includes:

   an integration constant deriving step of deriving an integration constant through a time-averaged pressure to flow rate ratio derived from the mathematical model of the vascular system;
   a blood pressure function obtaining step of obtaining a blood pressure function by substituting the integration constant derived from the time-averaged pressure to flow rate ratio into an equation solution;
   a parameter equation obtaining step of obtaining parameter equations by substituting the integration constant into the blood pressure functions of the boundary conditions; and
   a parameter obtaining step of obtaining lumped parameters of the mathematical model of the vascular system from the parameter equations.

5. The method of any one of claims 1 to 4, wherein the parameter computing step includes an admittance function expression deriving step of deriving an admittance function expression of a blood vessel by applying the mathematical model of the vascular system to 3D vascular image data after the parameter computing step.

6. The method of claim 5, further comprising:

   after the admittance function expression deriving step,
   a parameter applying step of applying the lumped parameters to the vascular admittance function expression; and
   an average flow rate ratio set calculating step of calculating all cases of the average flow rate ratio set over time.

7. The method of claim 6, further comprising:
   a flow rate distribution ratio determining step of determining a value for a flow rate distribution ratio that maximizes the admittance function expression after the average flow rate ratio set calculating step.

8. The method of claim 7, further comprising:
   an outlet pressure function deriving step of deriving an outlet pressure function after the flow rate distribution ratio determining step.

9

9. The method of any one of claims 6 to 8, wherein the average flow rate ratio set calculating step includes a time-averaged flow rate ratio set updating step of correcting the time-averaged flow rate ratio set when a convergence condition of the average flow rate ratio set over time is not satisfied in the average flow rate ratio set calculating step, and

the time-averaged flow rate ratio set updating step repeatedly performs the flow rate function deriving step, the parameter obtaining step, the parameter computing step, the admittance function expression deriving step, the parameter applying step, and the average flow rate ratio set calculating step until the flow rate distribution ratio of the average flow rate ratio set over time is determined.

10. An apparatus for calculating and obtaining outlet boundary conditions of a blood vessel of an examinee, the apparatus comprising:

a processor; and
a memory storing a computer program that is executed in the processor,
wherein the computer program:

obtains blood flow information by being provided with 3D vascular image data and biological information of the examinee and derives a blood flow equation by inputting the blood flow information as input values; and derives a flow rate function for an inflow boundary over time from the blood flow information, obtains lumped parameters of a mathematical model of a vascular system by computing simultaneous equations for blood pressure, and computes the admittance using an equation that defines the magnitude of peripheral admittance.

11. The apparatus of claim 10, wherein an admittance function expression of a blood vessel is derived by applying the mathematical model of the vascular system to the 3D vascular image data after the lumped parameters are computed, and the lumped parameters are applied to the admittance function expression of the blood vessel; and all cases of the average flow rate ratio set over time are calculated, a value for a flow rate distribution ratio that maximizes the admittance function expression is calculated, and an outlet pressure function is derived.

12. The apparatus of claim 10 or 11, wherein when a convergence condition of the average flow rate ratio set over time is not satisfied, lumped parameters and an admittance function expression are derived by deriving a flow rate function until a convergence condition of the average flow rate ratio set over time is satisfied by correcting the time-averaged flow rate ratio set, and calculation of an average flow rate ratio set is repeatedly performed by applying the derived parameters.

FIG. 1

FIG. 2

FIG. 3

S5000

```
           ┌──────────┐
           │  START   │
           └────┬─────┘
                ↓                           S100
     ┌──────────────────────┐
     │ BIOLOGICAL INFORMATION│
     │    PROVIDING STEP     │
     └──────────┬───────────┘
                ↓                           S200
     ┌──────────────────────┐
     │ BLOOD FLOW INFORMATION│
     │    INPUTTING STEP     │
     └──────────┬───────────┘
                ↓                           S300
     ┌──────────────────────┐
     │  BLOOD FLOW EQUATION  │
     │    DERIVING STEP      │
     └──────────┬───────────┘
                ↓                           S400
     ┌──────────────────────┐          ┌──────────────────┐   S910
     │  FLOW RATE FUNCTION   │←─────────│  TIME-AVERAGED   │
     │    DERIVING STEP      │          │ FLOW RATE RATIO  │
     └──────────┬───────────┘          │ SET UPDATING STEP│
                ↓              S500     └────────┬─────────┘
     ┌──────────────────────┐
     │      PARAMETER        │
     │    OBTANING STEP      │
     └──────────┬───────────┘
                ↓              S600
     ┌──────────────────────┐
     │PARAMETER COMPUTING STEP│
     └──────────┬───────────┘
                ↓              S700
     ┌──────────────────────┐
     │ ADMITTANCE FUNCTION   │
     │EXPRESSION DERIVING STEP│
     └──────────┬───────────┘      S800
                ↓                        ◇──────────────◇   S900
     ┌──────────────────────┐         ╱ AVERAGE FLOW     ╲
     │PARAMETER APPLYING STEP│───────→  RATE RATIO SET
     └──────────────────────┘         ╲ CALCURATING STEP ╱
                                        ◇──────┬───────◇
                                               ↓            S1000
                                   ┌──────────────────────┐
                                   │ FLOW RATE DISTRIBUTION│
                                   │ RATIO DETERMINING STEP│
                                   └──────────┬───────────┘
                                              ↓             S2000
                                   ┌──────────────────────┐
                                   │ OULET PRESSURE FUNCTION│
                                   │    DERIVING STEP      │
                                   └──────────────────────┘
```

FIG. 4

S300

EQUATION SOLUTION DERIVING STEP — S310

BOUNDARY CONDITION SETTING STEP — S320

BLOOD PRESSURE FUNCTION DERIVING STEP — S330

FIG. 5

S600

INTEGRATION CONSTANT DERIVING STEP — S610

BLOOD PRESSURE FUNCTION OBTAINING STEP — S620

PARAMETER EQUATION OPTAINING STEP — S630

PARAMETER OBTAINING STEP — S640

FIG. 6

FIG. 7

| | Europäisches Patentamt European Patent Office Office européen des brevets | **EUROPEAN SEARCH REPORT** | **Application Number** EP 25 15 0813 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SARABIAN MOHAMMAD ET AL: "Physics-Informed Neural Networks for Brain Hemodynamic Predictions Using Medical Imaging", IEEE TRANSACTIONS ON MEDICAL IMAGING, vol. 41, no. 9, 23 March 2022 (2022-03-23), pages 2285-2303, XP093232263, USA ISSN: 0278-0062, DOI: 10.1109/TMI.2022.3161653 | 1-3,5,10 | INV. G16H50/50 G16H30/40 |
| A | * abstract; p.3, first par.; p.4, col.1, par.2; p.8, col.1, par.E.1; p.8, col.2, par.1, 2; figure 2; figure 4 * | 4,6-9, 11,12 | |
| X | YU HUIDAN ET AL: "Inlet and Outlet Boundary Conditions and Uncertainty Quantification in Volumetric Lattice Boltzmann Method for Image-Based Computational Hemodynamics", FLUIDS, vol. 7, no. 1, 10 January 2022 (2022-01-10), pages 1-15, XP093273542, ISSN: 2311-5521, DOI: 10.3390/fluids7010030 Retrieved from the Internet: URL:https://doi.org/10.3390/fluids7010030> | 1-7,10 | TECHNICAL FIELDS SEARCHED (IPC) G16H |
| A | * abstract; p.2, last par.; p.4, par. 2.1, p.5, last par.; p.6, second par.; p. 6, last par. - p.7, first par.; figure 3 * | 8,9,11, 12 | |

-/--

The present search report has been drawn up for all claims

| Place of search | Date of completion of the search | Examiner |
|---|---|---|
| Munich | 5 May 2025 | Sundqvist, Benjamin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 25 15 0813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | CHOI, JAE HYUN ET AL: "Maximum admittance method for cerebrovascular outlet boundary conditions and importance of stenosis severity as a dominant factor on hemodynamics ¦ Scientific Reports", SCIENTIFIC REPORTS, vol. 15, no. 1, 3 April 2025 (2025-04-03), pages 1-49, XP093273304, US ISSN: 2045-2322, DOI: 10.1038/s41598-025-90604-0 Retrieved from the Internet: URL:https://www.nature.com/articles/s41598-025-90604-0> * the whole document * | 1-12 | |

TECHNICAL FIELDS
SEARCHED (IPC)

The present search report has been drawn up for all claims

| Place of search | Date of completion of the search | Examiner |
|---|---|---|
| Munich | 5 May 2025 | Sundqvist, Benjamin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

**REFERENCES CITED IN THE DESCRIPTION**

**Patent documents cited in the description**

- KR 1020240003981

- KR 102099951